(19) Europäisches Patentamt
European Patent Office
Office européen des brevets

(11) Veröffentlichungsnummer: 0 181 445 A2

# (12) EUROPÄISCHE PATENTANMELDUNG

(21) Anmeldenummer: 85110478.6

(22) Anmeldetag: 21.08.85

(51) Int. Cl.[4]: C 07 D 307/93

(30) Priorität: 21.09.84 DE 3434613

(43) Veröffentlichungstag der Anmeldung: 21.05.86 Patentblatt 86/21

(84) Benannte Vertragsstaaten: DE FR GB NL

(71) Anmelder: DYNAMIT NOBEL AKTIENGESELLSCHAFT
Postfach 1261
D-5210 Troisdorf, Bez. Köln(DE)

(72) Erfinder: Schmidt, Hans-Georg, Dr.
Porzer Strasse 15
D-5216 Niederkassel 3(DE)

## (54) Verfahren zur Herstellung des Lactons des Hemiacetals der cis-3,3-Dimethyl-2-formyl-cyclopropan-1-carbonsäure.

(57) Die vorliegende Erfindung beschreibt ein neues Verfahren zur Herstellung eines Lactons des Hemiacetals der cis-3,3-Dimethyl-2-formyl-cyclopropan-1-carbonsäure, bei dem bisher nicht bekannte Substanzen als Ausgangsprodukte verwendet werden. Die als Ausgangsprodukte eingesetzten Verbindungen sind Derivate des Valerolactons, die in 6-Stellung entweder durch einen Alkoxyrest oder durch einen Pyridiniumrest ersetzt sind. Außerdem sind diese Derivate in 4-Stellung durch zwei Methylgruppen und in 5-Stellung durch Halogen, vorzugsweise Chlor, substituiert. Das beanspruchte Verfahren besteht darin, diese Derivate des Valerolactons mit einer Base im Temperaturbereich zwischen −20 und + 100° C umzusetzen. Als Base wird vorzugsweise ein Alkalialkoholat eingesetzt.

EP 0 181 445 A2

Troisdorf, den 17.09.1984
OZ 84048 Dr.Sk/br.

DYNAMIT NOBEL AKTIENGESELLSCHAFT
5210 Troisdorf

# Verfahren zur Herstellung des Lactons des Hemiacetals der cis-3,3-Dimethyl-2-formyl-cyclopropan-1-carbonsäure

Gegenstand der vorliegenden Erfindung ist ein neues Verfahren zur Herstellung des Lactons des Hemiacetals der cis-3,3-Dimethyl-2-formyl-cyclopropan-1-carbonsäure mit Ausgangsprodukten, die bisher nicht zur Herstellung dieser Substanz verwendet wurden.

Es sind mehrere Verfahren bekannt, Lactone von Hemiacetalen der cis-3,3-Dimethyl-cyclopropan-1-carbonsäure herzustellen. Zusammenfassend werden diese Verfahren z.B. in "Chemistry and Industry" (1984), Seite 199 ff. beschrieben. Nachteilig an diesen bekannten Verfahren ist der Umstand, daß für ihre technische Durchführung schwer zugängliche Ausgangsprodukte eingesetzt werden müssen oder daß ihre Durchführung mit einem hohen technischen Aufwand, wie z.B. beim Einsatz von Butyllithium oder der notwendigen Miteinbeziehung einer Ozonolysestufe, erfolgen muß.

1 Es bestand deshalb die Aufgabe, ein neues Verfahren zur Herstellung der genannten Verbindung aufzufinden, das von gut herstellbaren Verbindungen ausgeht und sich ohne großen technischen Aufwand durchführen läßt.

In Erfüllung dieser Aufgabe wurde nun ein Verfahren zur Herstellung der Lactone des Hemiacetals der cis-3,3-Dimethyl-2-formyl-cyclopropan-1-carbonsäure der Formel I

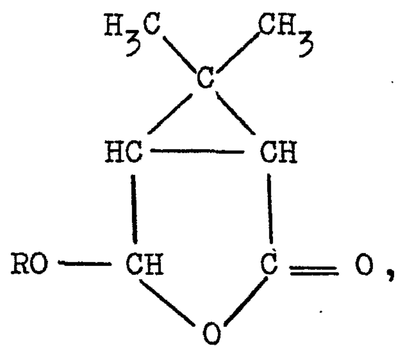

(I)

in der R für einen Alkylrest steht, gefunden, das durch die im Anspruch 1 genannten Maßnahmen gekennzeichnet ist.

Die als Ausgangsprodukte für das neue Verfahren einsetzbaren Derivate des Valerolactons sind leicht herstellbare Produkte, und das beanspruchte Verfahren zeichnet sich im Gegensatz zu den erwähnten bekannten Verfahren durch einfache Durchführungsformen aus.

Für den Fall, daß als Ausgangsprodukt ein Valerolacton eingesetzt wird, bei dem der Rest R' ein Alkoxyrest ist, kann als Base eine beliebige Base eingesetzt werden. Vorzugsweise wird jedoch ein Alkalialkoholat, wie z.B. Natrium- oder Kaliummethylat oder Kalium-tertiär-butylat, verwendet werden. Die Menge der Base liegt vorzugsweise zwischen 0,8 und 1,2 Moläquivalenten, bezogen auf das substituierte Valerolacton der Formel II.

Das Valerolacton der Formel II ist eine leicht zugängliche Verbindung, die z.B. durch Umsetzung von Dihalogenvalerolactonen der Formel

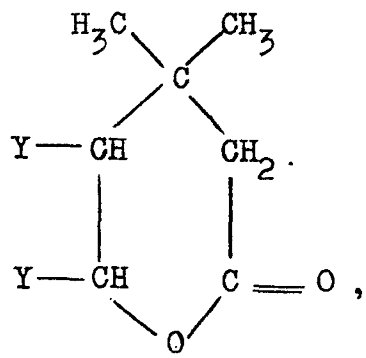

in der Y gleich Cl oder Br bedeutet, mit einem Alkoholat bei Temperaturen zwischen - 10 und 50° C erhalten werden. Der Alkylrest des Alkoholats hat vorzugsweise 1 bis 6 C-Atome. Die Reaktion wird vorzugsweise in Gegenwart eines aprotischen Lösungsmittels, wie z.B. aromatischen oder aliphatischen Kohlenwasserstoffen, durchgeführt. Die Herstellung des Dihalogenvalerolactons, das auch als 4,4-Dimethyl-5,6-tetrahydro-α-pyron bezeichnet wird, wird in der EP-A2 31 932 beschrieben.

Wenn als Ausgangsprodukt für das beanspruchte Verfahren ein Valerolacton verwendet wird, bei dem der Rest R' für 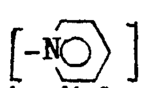 $^{+}Cl^{-}$ steht, muß die Base zu dem Valerolacton im Molverhältnis 1,8 bis 2,2 : 1 eingesetzt werden, wobei mindestens ein Moläquivalent der Base ein Alkalialkoholat sein muß. Vorzugsweise wird deshalb die gesamte einzusetzende Menge der Base in Form eines Alkalialkoholats eingesetzt. Prinzipiell ist es jedoch auch möglich, die über ein Moläquivalent liegende Menge der Base in Form einer anderen basisch reagierenden Verbindung einzusetzen.

Bei dieser Durchführungsform bestimmt das eingesetzte Alkalialkoholat den Rest R der erhaltenen Verbindung der Formel I. Es können deshalb beliebige Alkalialkoholate eingesetzt werden, je nachdem, welches Zielprodukt erhalten werden soll. Die bevorzugten Alkoholate sind diejenigen mit 1 bis 6 C-Atomen in der Alkylgruppe des Alkoholatrestes.

Die als Ausgangsprodukte eingesetzten Pyridiniumsalze des Valerolactons lassen sich aus dem ebenfalls in der EP-A2 31 932 genannten 4,4-Dimethyl-3,4-dihydro-α-pyron in einfacher Weise durch Reaktion mit Chlor und anschließender Umsetzung des als Zwischenprodukts erhaltenen 3,3-Dimethyl-4-chlor-5-oxo-pentansäurechlorids mit Pyridin gewinnen. Dabei erfolgt die Chlorierung bei Temperaturen unterhalb von 0° C und die anschließende Umsetzung mit Pyridin ohne Isolierung des obengenannten Zwischenprodukts bei Temperaturen zwischen - 10 und + 80° C. Dabei fällt das Pyridiniumchlorid als kristalliner Niederschlag aus, wenn in einem inerten Lösungsmittel, wie z.B. einem aliphatischen Kohlenwasserstoff, gearbeitet wird.

Unabhängig davon, welche Ausgangsprodukte gewählt werden, wird die erfindungsgemäße Reaktion im Temperaturbereich zwischen - 20 und + 100° C durchgeführt. Der bevorzugte Temperaturbereich liegt zwischen 15 und 70° C.

Es ist empfehlenswert, die erfindungsgemäße Reaktion in einem bei der Reaktion inerten Lösungsmittel durchzuführen. Dazu eignen sich hauptsächlich aprotische Lösungsmittel, wie aliphatische oder aromatische Kohlenwasserstoffe, Ether oder Nitrile. Als Beispiele seien genannt: Hexan, Heptan, Octan, Benzol, Toluol, Acetonitril oder Dimethylformamid.

Bei der erfindungsgemäßen Reaktion wird praktisch quantitativ das cis-Isomere des Lactons des Hemiacetals der 3,3-Dimethyl-2-formyl-cyclopropan-1-carbonsäure erhalten. Diese Verbindung wird in der Literatur auch als cis-3,3-Dimethyl-2-formyl-cyclopropancarbonsäure (Lacton-monoalkylacetal) bezeichnet. Dabei kann der Alkylrest sowohl geradkettig als auch verzweigt oder cyclisch sein, je nachdem, welche Ausgangsprodukte eingesetzt werden. Vorzugsweise besitzt der Alkylrest 1 bis 6 C-Atome.

Die erfindungsgemäß erhaltenen Hemiacetale der cis-3,3-Dimethyl-2-formyl-cyclopropan-1-carbonsäure sind wichtige Zwischenprodukte zur Herstellung von Pyrethroiden, die eine Cyclopropanstruktur enthalten. Ein solches Pyrethroid ist z.B. das Decamethrin, das zu den wirksamsten Insektiziden auf dieser Basis zählt.

Beispiel 1

16 g 4,4-Dimethyl-5-chlor-6-methoxy-tetrahydro-$\alpha$-pyron werden in 30 g Toluol gelöst und zu dieser Mischung unter Rühren bei 20° C 4,4 g $NaOCH_3$ zudosiert. Danach wird 3,5 Std. bei 50° C gerührt. Das bei der Umsetzung sich bildende Kochsalz wird abfiltriert und daraufhin der Rückstand destilliert. Man erhält 9,3 g cis-3,3-Dimethyl-2-formyl-cyclopropancarbonsäure (Lacton-monomethylacetal). Sdp. 59° C/0,3 mm.
Die Struktur wird durch NMR- und MS-Analyse bestätigt.

Beispiel 2

19 g 4,4-Dimethyl-5-brom-6-methoxy-tetrahydro-$\alpha$-pyron werden mit 4,4 g $NaOCH_3$ analog zu Beispiel 1 umgesetzt. Man erhält 8 g cis-3,3-Dimethyl-2-formyl-cyclopropancarbonsäure (Lacton-monomethylacetal).

Beispiel 3

Es werden 5 g des Pyridiniumchlorids des 4,4-Dimethyl-5,6-dichlor-tetrahydro-α-pyrons (Verbindung II mit R' = 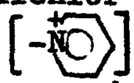 $Cl^-$) in 30 ml Acetonitril gelöst. Zu dieser Lösung werden unter Rühren 2,0 g $NaOCH_3$ zudosiert. Danach wird 8 Std. auf 50° C erhitzt. Danach wird der entstandene Feststoff abfiltriert und aus dem Filtrat das Lösungsmittel im Vakuum entfernt. Der Rückstand beträgt 2,2 g und besteht lt. NMR-Analyse aus cis-3,3-Dimethyl-2-formyl-cyclopropancarbonsäure (Lactonmonomethylacetal).

Troisdorf, den 17.09.1984
OZ 84048 Dr.Sk/br.

Patentansprüche

1. Verfahren zur Herstellung eines Lactons des Hemiacetals der cis-3,3-Dimethyl-2-formyl-cyclopropan-1-carbonsäure der Formel I

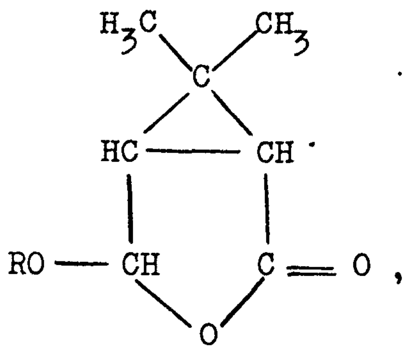

(I)

in der R für einen Alkylrest steht, dadurch gekennzeichnet, daß man ein δ-Valerolacton der Formel II

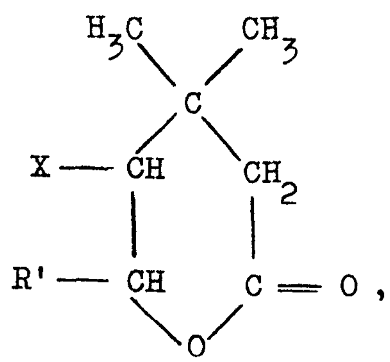

(II)

in der R' ein Alkoxyrest ist oder für den Rest 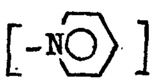 $^{+}Cl^{-}$ und X für Cl oder Br oder nur für Cl im Falle, daß R gleich 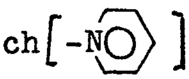 $^{+}Cl^{-}$ ist, steht, mit einer Base umsetzt.

2. Verfahren gemäß Anspruch 1, dadurch gekennzeichnet, daß als Base ein Alkalialkoholat eingesetzt wird.

3. Verfahren gemäß Anspruch 1 oder 2, dadurch gekennzeichnet, daß die Umsetzung in einem inerten Lösungsmittel durchgeführt wird.